# EUROPÄISCHE PATENTANMELDUNG

(11) **EP 1 850 119 A1**
(43) Veröffentlichungstag der Anmeldung: **31.10.2007**
(21) Anmeldenummer: 07105607.1
(22) Anmeldetag: 04.04.2007
(51) Int. Cl.: G01N 21/896, G01N 33/38, G02B 21/00

(54) **Optischer Sensor und Verfahren zur optischen Inspektion von Oberflächen**

(30) Priorität: 26.04.2006 DE 102006019468
(71) Anmelder: SIEMENS AKTIENGESELLSCHAFT, 80333 München (DE)
(72) Erfinder: Stockmann, Michael, 83052, Bruckmühl (DE); Weber, Martin, 85570, Markt Schwaben (DE); Heine, Wolfgang, 82024, Taufkirchen (DE); Spriegel, Dieter, 81379, München (DE)

(57) **Zusammenfassung**

Ein Optischer Sensor wird zur Erfassung von punktförmigen, linienförmigen oder flächigen Defekten eingesetzt, die an glatten Oberflächen wie Glas auftreten können. Der Sensor bestehend aus:
a) einem telezentrischen Laserscanner (12) mit
- einem Laser (1) zur annähernd senkrechten Beleuchtung einer glatten Oberfläche (5),
- einem Scannspiegel (2),
- einer telezentrischen Optik (4) zur Führung von Beleuchtungs- und Detektionsstrahlen,

b) einer Erfassungseinheit (11) mit
- einer Detektoroptik (8),
- einer zentralen Blende (9), die konzentrisch in der Nähe der Detektoroptik von dieser aus in Richtung auf den telezentrischen Laserscanner (4) positioniert ist,
- einem hochempfindlichen Photomultiplier (6) zur Erfassung von Streulicht, das von Defekten auf glatten Oberflächen (5) ausgeht,
- einer dem Photomultiplier (6) vorgeschalteten Schlitzblende (7).

Anwendung/Produkt: Prüfanlage zur optischen Inspektion von Oberflächen

## Beschreibung

Die Erfindung betrifft ein Verfahren und eine Anordnung zur Detektion von Defekten auf glatten Oberflächen, wobei Defekte mit Abmessungen im Bereich von weniger als 1 µm behandelt werden.

Monitore beziehungsweise Fernsehgeräte mit Kathodenstrahlröhren werden zunehmend durch Flachbildschirme vom Markt verdrängt. Das für derartige Flachbildschirme verwendete Flachglas muss besonderen Anforderungen entsprechen und muss insbesondere defektfrei sein, wobei beide Oberflächenseiten einer Flachglasscheibe zu betrachten sind. Dabei ist die Glasseite, die mit elektrischen Anschlüssen versehen wird, also photolithographisch bearbeitet sein kann, mit anderen Anforderungen zu prüfen, als die nach außen gerichtete Seite der Flachglasscheibe. Insgesamt ist eine vollständige Kontrolle der Fertigung eines dünnen Flachglases notwendig, da auch extrem kleine Defekte die Prozessierung der Glasoberfläche beeinflussen und somit die Funktion des Monitors beeinträchtigen können. Bei der Fertigung von Flachglas kann standardmäßig ein hoher Durchsatz vorliegen, so dass Inspektions- oder Kontrollsysteme diese große Menge von Flachglas testen müssen, wobei die entsprechende Prüfung die gesamte Oberfläche abdeckt. Die häufigsten Defekte sind punkt- oder linienförmig. Da Defekte auch im Submikrometerbereich auszuschließen sind, müssen diese durch entsprechende Inspektionsverfahren erfasst und lokalisiert werden.

Zur Erkennung von punkt-, linien- und flächenförmigen Defekten werden meist Zeilenkameras mit geeigneter Beleuchtung eingesetzt. Die mit dieser Technologie erkennbaren Defekte liegen im Bereich von wenigen Mikrometern. Defekte im Submikrometerbereich wie beispielsweise extrem kleine Kratzer mit einer Tiefe von wenigen Nanometern können derzeit nur visuell von geschultem Personal mittels lichtstarker Lampen erfasst werden. Dieses Verfahren lässt sich allerdings nicht für eine hundertprozentige Kontrolle am Flachglas im Produktionstakt anwenden.

Der Erfindung liegt die Aufgabe zugrunde, eine Vorrichtung und ein Verfahren bereitzustellen, mittels derer die vollständige optische Prüfung von glatten Oberflächen bezüglich punkt-, linien- oder flächenförmiger Defekte möglich ist, wobei deren Abmaße im Submikrometerbereich liegen. Die Lösung dieser Aufgabe geschieht durch die Merkmalskombination der Ansprüche 1 beziehungsweise 10. Vorteilhafte Ausgestaltungen sind den Unteransprüchen zu entnehmen.

Der Erfindung liegt die Erkenntnis zugrunde, dass eine hundertprozentige Prüfung von glatten Oberflächen, insbesondere von Flachglas, auf Defekte, die punktförmig, flächenförmig oder linienförmig sein können und deren Abmessungen im Submikrometerbereich liegen, durch den Einsatz eines telezentrischen Laserscanners in Kombination mit einem Photomultiplier zur Detektion des an den Defekten gestreuten Laserlichtes ermöglicht wird. Eine sehr hohe Detektionsempfindlichkeit des Photomultipliers ermöglicht den Nachweis von Defekten im Submikrometerbereich bei gleichzeitig hoher Datenrate. Hierdurch wird insgesamt eine vollständige Endkontrolle von glatten Oberflächen beziehungsweise von Flachglas möglich. Sowohl der scannende Beleuchtungsstrahl als auch der Detektionsstrahlengang sind telezentrisch angeordnet. Dies erlaubt, das von einem Defekt ausgehende Streulicht über eine zur Beleuchtungsrichtung, die gleich der Messrichtung ist, rotationssymmetrische Apertur aufzufangen, wodurch insbesondere feine Kratzer unabhängig von ihrer Lage auf einer Oberfläche mit gleichmä-ßiger Empfindlichkeit betrachtet über die Fläche erkannt werden. Die optische Konstruktion des Sensors ist sehr einfach, da die Auflösung des Sensors nur vom Fokusdurchmesser des Scannstrahles bestimmt wird. Die Abbildungsgüte der Scannoptik muss aus diesem Grund lediglich an die im Vergleich zum Detektionsstrahlengangs geringe Apertur des Scannstrahles angepasst werden. Über eine Detektionsoptik wird das zurück gestreute Licht gesammelt und die Abbildungsgüte der Detektionsoptik unterliegt keinen besonderen Anforderungen.

Für die Erkennung von Kratzern ist es besonders vorteilhaft, die Apertur einer Detektoroptik rotationssysmmetrisch zur optischen Achse auszubilden. Da durch die telezentrische Ausbildung des Sensors für die Beleuchtung als auch für die Detektionsstrahlführung ein wesentlicher Beleuchtungslichtanteil an der zu prüfenden Oberfläche spiegelnd reflektiert wird, muss eine zentrale Blende eingesetzt werden, um derartige Lichtanteile nicht zum Photomultiplier gelangen zu lassen. Dies kann entweder durch eine Blende geschehen oder durch einen Umlenkspiegel, der in einer Variante des Sensors notwendig ist, indem der scannende Lichtstrahl von außen in den telezentrischen Strahlengang von außen zugeführt beziehungsweise eingekoppelt wird.

Eine derartige Blende im Zentrum der Detektoroptik blockiert das von beispielsweise einer Glasprobenoberfläche spiegelnd reflektierte Licht, das aufgrund der telezentrischen Beleuchtung auf den Beleuchtungsweg zurück laufen würde. Die Größe der hier vorgesehenen Blende erlaubt eine gewisse Winkeltoleranz der Glasprobe zur senkrechten Beleuchtungsrichtung. Durch die zentrale Blende wird eine ringförmige, also zur optischen Achse rotationssymmetrische Ausbildung der Detektorapertur realisiert, unabhängig vom Ort des Scannstrahles. Der Vorteil dieser ringförmigen Apertur ermöglicht gleichzeitig eine gleichmäßige Detektionsempfindlichkeit für linienförmige Defekte unabhängig von deren Richtung auf der Messoberfläche.

Falls die Detektionsapertur sich nicht ringförmig ausbilden lässt, sondern beispielsweise aus zwei gegenüber liegenden Kreisabschnitten besteht, kann in vorteilhafter Weise eine entstehende Winkellücke im Detektionsbereich mit dem Einsatz von zusätzlichen Sensoren überbrückt werden, wobei die Scannlinien der eingesetzten Sensoren jeweils gegeneinander geneigt sein müssen.

Ein beschriebener Sensor kann vorteilhaft bei der Inspektion von Flachglasscheiben eingesetzt werden, wobei Flachglasscheiben beispielsweise eine Materialstärke im zehntel Millimeterbereich aufweisen können und bis zu mehreren Quadratmetern groß sein können. Zum einen wird eine vollflächige Kontrolle des Flachglases nach dessen Herstellung ermöglicht. Weiterhin können punkt-, flächen- oder linienförmige Defekte im Submikrometerbereich erkannt werden. Mit einer Variante dieses Sensors, dessen Schärfentiefe deutlich geringer als die Dicke des zu untersuchenden Glases ist, kann zusätzlich festgestellt werden, ob ein Defekt auf der Oberseite oder auf der Unterseite des bei der Messung durchleuchteten Flachglases positioniert ist.

Um eine Anpassung an die bei der Produktion von Flachglas vorliegenden Vorschubgeschwindigkeiten zu erhalten, so dass die Prüfung zeitgleich beziehungsweise genauso schnell wie die Produktionsrate ablaufen kann, muss eine hohe Datenrate im Sensor zur Verfügung stehen. Dies wird durch den Einsatz eines hochempfindlichen Photomultipliers ermöglicht.

Im Folgenden werden anhand von schematischen, die Erfindung nicht einschränkenden Figuren Ausführungsbeispiele beschrieben.
- Figur 1: zeigt den prinzipiellen Aufbau eines Sensors mit Erfassungseinheit und telezentrischem Laserscanner,
- Figur 2: zeigt eine Variante eines Sensors entsprechend Figur 1, wobei eine um 90° um die optische Achse der Linsen gedrehte Darstellung gewählt ist.

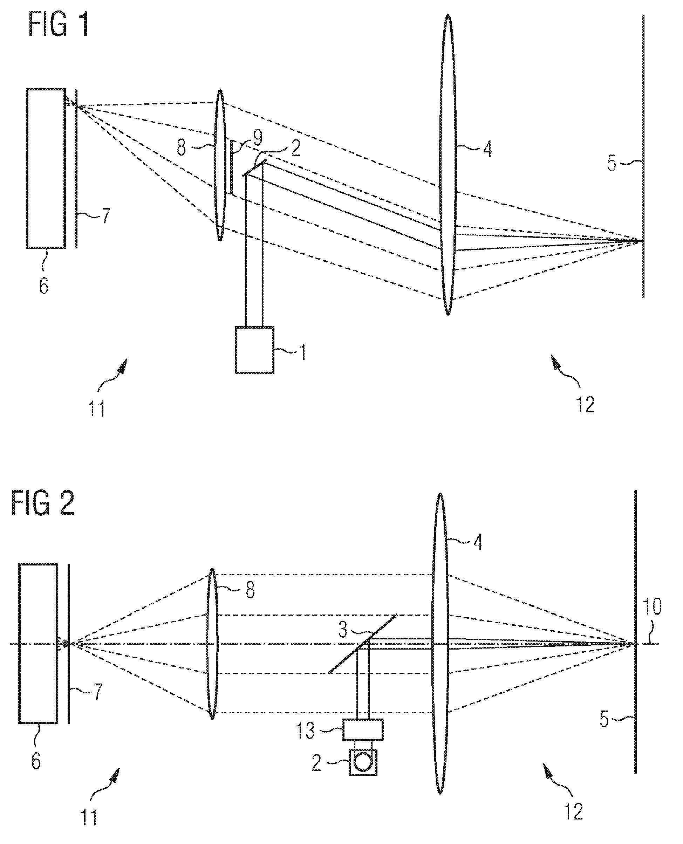

Figur 1, die den prinzipiellen Aufbau eines beschriebenen Sensors wiedergibt, zeigt, dass das kollimierte Licht eines Lasers von einem Scanner in einem bestimmen Winkelbereich abgelenkt wird. Ein Laser 1 generiert einen Laserstrahl, der mittels eines Scannspiegels 2 abgelenkt wird. Eine telezentrische Scannoptik 4 fokussiert das abgelenkte Licht auf die zu untersuchende glatte Oberfläche 5 beziehungsweise auf eine Glasprobe. Die Lichtstrahlen treffen senkrecht auf die Oberfläche, durch die Ablenkung des Scannspiegels beschreibt der Laserfokus auf der Oberfläche eine Linie. Die flächige Abtastung der Glasprobe wird durch den Vorschub der Probe senkrecht zur Scannlinie erreicht. Das von der Glasoberfläche reflektierte Streulicht wird von der telezentrischen Scannoptik 4 kollimiert und mit einer nachfolgenden Detektoroptik 8 auf eine Schlitzblende 7 fokussiert. Das fokussierte Licht durchläuft die Schlitzblende 7 und fällt auf die Empfangsfläche eines Photomultipliers 6. Die vor dem Photomultiplier 6 angeordnete Schlitzblende 7 schirmt diesen weitgehend vor Fremdlicht ab, welches nicht vom Ort des Laserfokus auf der Glasoberfläche ausgeht. Die Blende 9 im Zentrum der Detektoroptik blockiert das von der Glasoberfläche spiegelnd reflektierte Licht, welches aufgrund der telezentrischen Beleuchtung auf dem Detektionsstrahlweg zurück, in Figur 1 nach links, verläuft.

Mit der Größe der zentralen Blende 9, die in Grenzen variabel ausgestaltet sein kann, wird eine gewisse Unabhängigkeit des Sensors gegenüber Winkelabweichungen erzeugt, so dass die zu inspizierende Oberfläche in Grenzen zur senkrechten Beleuchtungsrichtung geneigt sein kann. Die zentrale Blende der Detektoroptik bewirkt eine ringförmige Detektorapertur unabhängig vom Ort des Scannstrahls. Diese ringförmige Apertur ermöglicht eine gleichmäßige Detektionsempfindlichkeit für linienförmige Defekte, unabhängig von deren Richtung auf der zu prüfenden Oberfläche.

Figur 2 zeigt eine Variante von der Darstellung in Figur 1, wobei hier eine um 90° um die optische Achse der Linsen gedrehte Anordnung gewählt ist. Die Scannoptik 4 besteht in diesem Beispiel aus einer weiteren Optik, um beispielsweise den Scannwinkel zu vergrößern. Da diese optische Komponente nicht vom reflektierten Streulicht durchstrahlt werden soll, muss sie außerhalb des Detektionsstrahlenganges angeordnet sein. Sie wird deshalb über einen Umlenkspiegel 3 zugeführt. Dieser Umlenkspiegel ersetzt zugleich die zentrale Blende 9, die in Figur 1 sichtbar ist. Dabei ist der Umlenkspiegel 3 wesentlich breiter als die zentrale Blende 9. Folglich wird die Detektionsapertur in diesem Beispiel nicht mehr ringförmig sein, sondern aus zwei gegenüberliegenden Kreisabschnitten bestehen. Die damit hinfällige Rotationssymmetrie der Apertur führt beispielsweise dazu, dass linienförmige Defekte nicht unter allen Umständen deutlich erkennbar sind. Diese Winkellücke im Detektionsbereich kann mit einem weiteren Sensor abgedeckt werden, dessen Scannlinie gegenüber der des ersten Sensors derart gedreht ist, dass sein Detektionsbereich die Winkellücke des ersten Sensors überdeckt.

Somit ist eine vollständige Kontrolle von Flachglas möglich und Defekte, die punkt-, flächen- oder linienförmig ausgebildet sind, können im Submikrometerbereich erkannt und lokalisiert werden. Insbesondere wird mit einem ersten Sensor gro-ßer Schärfentiefe die vollständige Prüfung der Oberfläche mit einer groben Lokalisierung der Defekte durchgeführt und mit einem zweiten Sensor geringer Schärfentiefe die Entscheidung vorbereitet, auf welcher Seite einer Flachglasscheibe der Defekt liegt.

Bei entsprechendem Vorschub einer Flachglasscheibe wird mit der oszillierenden Bewegung des Laserstrahles eine flächenhafte Abtastung des Objektes und somit die Erfassung der unterschiedlichen Defektarten erreicht.

Feine Kratzer streuen das sie beleuchtende Licht nur senkrecht zu ihrer Längsachse. Sie sind daher nur sichtbar, wenn sie senkrecht zu ihrer Längsachse beobachtet werden. Damit solche Kratzer unabhängig von ihrer Lage auf dem Glas erkannt werden können, muss die Apertur der Empfangsoptik rotationssymmetrisch zur Beleuchtungsrichtung angeordnet sein. Falls die Apertur aus technischen Gründen nicht vollständig rotationssymmetrisch ist, können mehrere Sensoren mit sich überlappenden Aperturbereichen verwendet werden. Dabei wird eine Objektoberfläche nacheinander gescannt. Die hohe Messgeschwindigkeit wird durch den parallelen Einsatz mehrerer Sensoren erreicht. Außerdem ermöglicht dieser angedeutete modulare Aufbau die Anpassung des Prüfsystems an unterschiedlich breite Glasplatten.

Da die Schärfentiefe des oben beschriebenen Sensors größer als die Glasdicke ist, können Defekte auf der Vorder- und auf der Rückseite zunächst nicht voneinander unterschieden werden. Hierzu ist ein zweiter Sensor vorgesehen, dessen Schärfentiefe kleiner als die Glasdicke ist und der somit Abstands- beziehungsweise Höhenwerte ausgeben kann. In der Praxis ist mit einem derartigen zweiten Sensor allein keine vollständige Kontrolle möglich. Daher wird dieser nur eingesetzt, wenn die laterale Lage eines Defektes, der mit dem ersten Sensor bereits gefunden wurde, fest steht und somit nur noch die Höhenlage bestimmt werden soll.

## Patentansprüche

1. Optischer Sensor zur Erfassung von punktförmigen, linienförmigen oder flächigen Defekten an glatten Oberflächen bestehend aus:
a) einem telezentrischen Laserscanner (12) mit
- einem Laser (1) zur annähernd senkrechten Beleuchtung einer glatten Oberfläche (5),
- einem Scannspiegel (2),
- einer telezentrischen Optik (4) zur Führung von Beleuchtungs- und Detektionsstrahlen,
b) einer Erfassungseinheit (11) mit
- einer Detektoroptik (8),
- einer zentralen Blende (9), die konzentrisch in der Nähe der Detektoroptik in Richtung auf den telezentrischen Laserscanner (4) positioniert ist,
- einem hochempfindlichen Photomultiplier (6) zur Erfassung von Streulicht, das von Defekten auf glatten Oberflächen (5) ausgeht,
- einer dem Photomultiplier (6) vorgeschalteten Schlitzblende (7) .

2. Optischer Sensor nach Anspruch 1, bei dem eine Apertur einer Empfangsoptik rotationssymmetrisch zur Beleuchtungsrichtung angeordnet ist.

3. Optischer Sensor nach Anspruch 2, bei dem eine Apertur einer Empfangsoptik ringförmig ausgebildet ist.

4. Optischer Sensor nach einem der vorhergehenden Ansprüche, bei dem bei einer nicht vollständig rotationssymmetrischen Ausbildung der Detektionsoptik (8), ein zweiter Sensor mit zum ersten Sensor überlappendem Aperturbereich vorhanden ist, dessen Scannlinie zur Scannlinie des ersten Sensors einen Winkel bildet.

5. Optischer Sensor nach einem der vorhergehenden Ansprüche, bei dem zur Vergrößerung eines Scannwinkels die Scannoptik (13) außerhalb des Detektionsstrahlenganges angeordnet ist und der scannende Laserstrahl über einen Umlenkspiegel (3) eingekoppelt wird, der gleichzeitig die zentrale Blende darstellt.

6. Optischer Sensor nach einem der vorhergehenden Ansprüche, bei dem die durch den Sensor abtastbare glatte Oberfläche eine Flachglasscheibe ist.

7. Optischer Sensor nach einem der vorhergehenden Ansprüche, bei dem der optische Sensor doppelt ausgeführt ist, um einerseits mit einer Fokustiefe, die beide Seiten einer Flachglasscheibe gleichzeitig erfasst, Defekte beidseitig lateral zu orten und andererseits mit einer Fokustiefe, die kleiner als die Materialstärke der Flachglasscheibe ist, die Höhenlage des Defektes und damit die den Defekt tragende Seite der Flachglasscheibe zu ermitteln.

8. Verfahren zur Erfassung von punktförmigen, linienförmigen oder flächigen Defekten auf glatten Oberflächen unter Einsatz eines optischen Sensors entsprechend einem der Ansprüche 1 bis 7, bei dem
- eine glatte Oberfläche (5) eines Objektes zu deren vollständiger Prüfung mittels eines telezentrischen Laserscanners (12) annähernd senkrecht beleuchtet wird,
- von einer Defektstelle ausgehendes Streulicht zur optisch/elektronischen Wandlung auf einen hochempfindlichen Photomultiplier (6) geführt wird,
- zur Ausschaltung von Fremdlicht dem Photomultiplier (6) eine Schlitzblende (7) vorgeschaltet ist, auf die das von einer Defektstelle ausgehende Streulicht fokussiert und von diesem durchlaufen wird, bevor es auf den Photomultiplier (6) trifft,
- zur Abschirmung von aufgrund des telezentrischen Aufbaues spiegelnd reflektierten Lichtes eine zentrale Blende (9) in dem Detektionsstrahlengang eingebracht wird,
- so dass sich eine ringförmige Apertur der Detektoroptik (8) mit gleichmäßiger Detektionsempfindlichkeit unabhängig von der Richtung des Defektes auf der Oberfläche ergibt.

9. Verfahren nach Anspruch 8, bei dem an transparentem Material von einer Seite beidseitig glatte Oberflächen vermessen werden.

10. Verfahren nach Anspruch 9, bei dem das zu vermessende transparente Material Flachglas ist.

11. Verfahren nach einem der Ansprüche 8 bis 10, bei dem ein erster und ein zweiter Sensor so ausgelegt werden, dass deren Beleuchtungsstrahlen unterschiedliche Fokusdurchmesser aufweisen, so dass sich jeweils eine Schärfentiefe ergibt, mit der zum einen die vollständige flächenhafte Erfassung von Defekten auf beiden Seiten des Flachglases möglich ist, und zum anderen die Position von zuvor mit dem ersten Sensor erkannten Defekten bezüglich Vorder- und Rückseite des Flachglases unterschieden werden kann.

12. Verfahren nach einem der Ansprüche 8 bis 11, bei dem zwei Sensorsysteme mit unterschiedlichen Schärfentiefen zeitversetzt verwendet werden, um zum einen die Gesamtheit aller Defekte zu erfassen und zum anderen die Höhenlage einzelner Defekte zu ermitteln.

13. Verfahren nach einem der Ansprüche 8 bis 12, bei dem eine vollflächige Abdeckung bei der Prüfung von flächigen Oberflächen durch den parallelen Einsatz mehrerer Sensoren bzw. mehrerer Sensorkombinationen erreicht wird.
